# EUROPÄISCHE PATENTSCHRIFT

(11) **EP 3 490 642 B1**
(45) Veröffentlichungstag und Bekanntmachung des Hinweises auf die Patenterteilung: **16.06.2021**
(21) Anmeldenummer: 17734224.3
(22) Anmeldetag: 21.06.2017
(51) Int. Cl.: A61M 5/20, A61M 5/24, A61M 5/315, A61M 5/50

(54) **VORRICHTUNG ZUR VERABREICHUNG EINER DOSIS EINES FLUIDEN PRODUKTS**
DEVICE FOR DELIVERY OF A DOSE OF A FLUID PRODUCT
DISPOSITIF D'ADMINISTRATION D'UNE DOSE D'UN PRODUIT FLUIDE

(30) Priorität: 26.07.2016 CH 9602016
(43) Veröffentlichungstag der Anmeldung: 05.06.2019
(73) Patentinhaber: Ypsomed AG, 3401 Burgdorf (CH)
(72) Erfinder: STAUB, Seline, 8604 Volketswil (CH); HIRSCHEL, Jürg, 3007 Bern (CH)
(74) Vertreter: Kleiner, Stefan
(86) Internationale Anmeldenummer: PCT/CH2017/000063
(87) Internationale Veröffentlichungsnummer: WO 2018/018166

(56) Entgegenhaltungen:
- WO-A1-2009/100550
- WO-A1-2015/055588
- WO-A2-2013/033850

## Beschreibung

Die Erfindung betrifft eine Vorrichtung zur Verabreichung einer Dosis eines fluiden Produkts.

Der Begriff "Medikament" umfasst hier jede fliessfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oligonukleotide, Antikörper oder Teile von Antikörpern sowie geeignete Basis-, Hilfs- und Trägerstoffe. Aus dem Stand der Technik sind solche Vorrichtungen bekannt. Beispielsweise aus WO 2009/100550 A1 ist eine Vorrichtung zur Verabreichung einer Dosis bekannt. Weitere relevante Beispiele aus dem Stand der Technik sind aus der WO2013/033850 sowie der WO2015/055588 bekannt.

Es ist eine Aufgabe der Erfindung eine alternative Vorrichtung zur Verabreichung einer Dosis bereitzustellen, wobei die Dosiergenauigkeit erhöht ist.

Diese Aufgabe wird durch eine Vorrichtung zur Verabreichung einer Dosis eines fluiden Produkts gemäss dem Patentanspruch 1 erfüllt. Bevorzugte Ausführungsformen einer solchen Vorrichtung gehen aus den abhängigen Patentansprüchen hervor.

Im Folgenden bedeutet die proximale Richtung bei einer Vorrichtung die Richtung zum gehäuseseitigen Ende hin und die distale Richtung bei einer Vorrichtung die Richtung zum nadelseitigen Ende hin.

Die Erfindung betrifft eine Vorrichtung zur Verabreichung einer Dosis eines fluiden Produkts mit einem Gehäuse, welches ein distales und ein proximales Ende oder Abschnitt aufweist. Ferner weist die Vorrichtung eine vorgespannte Feder auf, welche zumindest teilweise von einem Antriebsglied aufgenommen ist. Das Antriebsglied ist axial bewegbar in dem Gehäuse gelagert, um einen in einer Karpule, insbesondere um einen in einer Mehrkammerkarpule aufgenommenen Stopfen zur Verabreichung der Dosis des fluiden Produkts in die distale Richtung zu bewegen. Zur Begrenzung der Abgabe oder Ausschüttung einer Dosis aus der Vorrichtung ist an dem Gehäuse ein Ausschüttungsanschlag vorgesehen, welcher in Anschlagkontakt mit einem an dem Antriebsglied vorgesehenen Ausschüttungsanschlag gelangen kann. Besonders bevorzugt kann der Ausschüttungsanschlag des Antriebsglieds in axialen Anschlagkontakt mit dem Ausschüttungsanschlag des Gehäuses gelangen. Die abzugebende oder auszuschüttende Dosis kann vorzugsweise eine festgelegte Dosis sein. Die Vorrichtung umfasst ferner ein Halteelement zum Halten des mit einer Kraft der Feder beaufschlagten Antriebsglieds. Das Halteelement ist vorzugsweise drehfest relativ zu dem Gehäuse und/oder Antriebsglied angeordnet. Das Halteelement und/oder das Gehäuse können hülsenförmig ausgebildet sein. Das Halteelement kann zumindest teilweise von dem Gehäuse aufgenommen sein. Die Feder ist an dem distalen Ende an dem Antriebsglied und an dem proximalen Ende an dem Halteelement abgestützt. In einem Ausgangszustand der Vorrichtung, in welchem die Vorrichtung ausgeliefert wird, ist das Antriebsglied durch einen Eingriff zwischen einem an dem Halteelement vorgesehenen Eingriffselement und einem an dem Antriebsglied vorgesehenen Eingriffselement in einer proximalen Position haltbar, wobei in einem ausgelösten Zustand der Vorrichtung, wobei die Abgabe oder Ausschüttung der Dosis aus der Vorrichtung ausgelöst ist, das Eingriffselement des Halteelements ausser Eingriff mit dem Eingriffselement des Antriebsglieds ist. Zumindest in dem Ausgangszustand der Vorrichtung ist das Halteelement in proximaler Richtung von dem proximalen Ende oder Abschnitt des Gehäuses, insbesondere von einem proximalen Gehäuseanschlag, welcher an dem proximalen Ende oder Abschnitt des Gehäuses vorgesehen ist, beabstandet. Der proximale Gehäuseanschlag kann vorzugweise an einer Mantelinnenfläche des Gehäuses vorgesehen sein. Das Halteelement ist zumindest in dem Ausgangszustand relativ zu dem Gehäuse in die proximale Richtung verformbar und/oder ausdehnbar. In dem ausgelösten Zustand der Vorrichtung kann das Halteelement aufgrund der in die proximale Richtung wirkenden Kraft der Feder in proximaler Richtung in Anschlagkontakt mit dem proximalen Ende oder Abschnitt des Gehäuses, insbesondere an dem proximalen Gehäuseanschlag gelangen. Der Ausschüttungsanschlag des Antriebsglieds ist axial in die proximale Richtung versetzt zu dem Eingriffselement des Antriebsglieds an dem Antriebsglied angeordnet.

Durch die Anordnung und/oder Ausgestaltung der erfindungsgemässen Vorrichtung kann eine Feder mit einer grossen Kraft in die Vorrichtung aufgenommen werden oder ein fluides Produkt mit einer grossen Viskosität verabreicht werden oder eine grosse Dosismenge eines fluiden Produkts ausgeschüttet werden, ohne dass die Dosiergenauigkeit beeinträchtigt ist.

Besonders bevorzugt kann ferner der Ausschüttungsanschlag des Antriebsglieds in Umfangsrichtung um 90 Grad versetzt zu dem Eingriffselement des Antriebsglieds an dem Antriebsglied angeordnet sein. Das Antriebsglied kann vorzugswiese hülsenförmig ausgebildet sein. Das Antriebsglied ist derart ausgebildet, dass es zumindest teilweise die vorgespannte Feder aufnehmen kann. Das Halteelement ist derart ausgebildet, dass das Halteelement das Antriebsglied zumindest in dem Ausgangszustand teilweise aufnehmen kann.

Das proximale Ende des Antriebsglieds ist vorzugsweise in dem Ausgangszustand der Vorrichtung in Anschlagkontakt mit dem Gehäuse, insbesondere mit dem proximalen Ende oder Abschnitt des Gehäuses. Besonders bevorzugt ist das proximale Ende des Antriebsglieds in dem Ausgangszustand der Vorrichtung in Anschlagkontakt mit dem proximalen Gehäuseanschlag des Gehäuses. Ferner kann das Halteelement einen Steg aufweisen, welcher zumindest in dem Ausgangszustand der Vorrichtung in einer an dem proximalen Ende des Antriebsglieds vorgesehenen Kerbe angeordnet ist. Diese Anordnung kann dazu dienen, dass das Halteelement und das Antriebsglied relativ zueinander drehfest gelagert sind. Ferner kann diese Anordnung dazu dienen, dass das Haltelement in dem Ausgangszustand der Vorrichtung von dem proximalen Ende oder Abschnitt des Gehäuses, insbesondere von dem proximalen Gehäuseanschlag des Gehäuses beabstandet ist. Es sind aber auch andere Anordnungen und Ausgestaltung der Vorrichtung möglich, wobei das Haltelement in dem Ausgangszustand der Vorrichtung von dem proximalen Ende oder Abschnitt des Gehäuses, insbesondere von dem proximalen Gehäuseanschlag des Gehäuses beabstandet ist.

Das Eingriffselement des Halteelements kann vorzugsweise als Nocke ausgebildet sein. Das Halteelement kann mehrere Eingriffselemente aufweisen. Besonders bevorzugt umfasst das Halteelement zwei Eingriffselemente oder Nocken, welche jeweils an einem spannbaren Arm des Halteelements, insbesondere an einem radial nach aussen elastisch auslenkbaren beziehungsweise an einem radial nach innen elastisch einlenkbaren Arm des Halteelements angeordnet sind. Die Arme, insbesondere die zwei Arme des Halteelements können vorzugsweise über den Steg des Halteelements miteinander verbunden sein.

Das Eingriffselement des Antriebsglieds kann vorzugweise als Aussparung ausgebildet sein. Das Antriebsglied kann mehrere Eingriffselemente aufweisen. Besonders bevorzugt umfasst das Antriebsglied zwei Eingriffselemente oder Aussparungen.

Alternativ kann das Eingriffselement des Antriebsglieds als Nocke ausgebildet sein und das Eingriffselement des Halteelements als Aussparung ausgebildet sein.

Das Eingriffselement des Halteelements oder des Antriebsglied können auch eine andere Ausgestaltung aufweisen, sofern das Eingriffselement des Halteelements und das Eingriffselement des Antriebsglieds lösbar in Eingriff sein können. Vorzugsweise sind das Eingriffselement des Halteelements und/oder das Eingriffselement des Antriebsglied derart ausgebildet, dass eine Verformung und/oder Ausdehnung des Eingriffselements des Halteelements und/oder des Eingriffselements des Antriebsglied vermindert oder verhindert ist, wenn das Eingriffselement des Halteelements und/oder das Eingriffselements des Antriebsglied in Eingriff sind und eine Kraft der Feder auf das Antriebsglied und auf das Halteelement wirkt. Besonderes bevorzugt sind das Antriebsglied und/oder das Haltelement derart ausgebildet, dass eine Verformung und/oder Ausdehnung des Antriebsglied und/oder Haltelement vermindert oder verhindert ist, wenn eine Kraft der Feder auf das Antriebsglied und auf das Halteelement wirkt.

Ferner ist vorzugsweise der Aussendurchmesser des Antriebglieds am distalen Ende des Antriebsglieds und am proximalen Ende des Antriebsglieds unterschiedlich, wobei der Innendurchmesser des Antriebsglieds am distalen und am proximalen Ende des Antriebsglieds gleich ist. Die Grösse des Innendurchmessers des Antriebsglieds ist derart ausgewählt, dass die Feder zumindest teilweise von dem Antriebsglied aufgenommen werden kann. Besonders bevorzugt ist der Aussendurchmesser des Antriebsglieds grösser am proximalen Ende als am distalen Ende des Antriebsglieds, wobei der Aussendurchmesser des distalen Ende des Antriebsglieds derart ausgebildet ist, dass das distale Ende des Antriebsglieds in die Karpule, insbesondere in die Mehrkammerkarpule ragt, wenn der Ausschüttungsanschlag des Antriebsglieds in Anschlagkontakt mit dem Ausschüttungsanschlag des Gehäuses ist. Durch diese entsprechende Anpassung der Wanddicke des Antriebsglieds kann die dosisrelevante Ausdehnung beziehungsweise dosisrelevante Kompression des Antriebsglieds verringert oder sogar ausgeschlossen werden.

Das Halteelement kann ein Element zur Führung der Feder aufweisen. Die Feder ist vorzugsweise als Wendelfeder ausgebildet. Die wendelförmige Feder ist um das Element des Halteelements angeordnet.

Um die Vorrichtung auszulösen, weist die Vorrichtung vorzugsweise ein Betätigungselement auf, welches relativ zu dem Gehäuse bewegbar ist. Das Betätigungselement ist vorzugsweise relativ zu dem Gehäuse axial bewegbar angeordnet. Das Betätigungselement kann eine Ausnehmung umfassen, in welche das Eingriffselement des Halteelements in dem ausgelösten Zustand der Vorrichtung ragbar ist. Dabei gelangt das Eingriffselement des Halteelements ausser Eingriff mit dem Eingriffselement des Antriebsglieds. Das Antriebsglied ist folglich von dem Halteelement gelöst und kann sich aufgrund der Kraft der Feder relativ zu dem Gehäuse in die distale Richtung bewegen.

Die Vorrichtung umfasst ferner eine Aufnahmevorrichtung zur Aufnahme des fluiden Produkts, welche relativ zum Gehäuse drehbar oder schraubbar gelagert ist. Die Aufnahmevorrichtung kann eine Karpule, insbesondere eine Mehrkammerkarpule aufnehmen. Besonders bevorzugt kann die Aufnahmevorrichtung eine Mehrkammerkarpule aufnehmen, wobei die Mehrkammerkarpule durch Drehung der Aufnahmevorrichtung relativ zu dem Gehäuse abmischbar ist. Die Mehrkammerkarpule kann vorzugsweise als 2-Kammerkarpulen mit zwei Stopfen und einem Bypass ausgebildet sein. Damit die Mehrkammerkarpule, insbesondere die 2-Kammerkarpule in einer definierten Position relativ zu der Aufnahmevorrichtung gehalten werden kann, ist vorzugsweise an einem distalen Ende des Antriebsglieds ein Haltearm, insbesondere zwei Haltearme vorgesehen. Die Haltearme des Antriebsglieds halten die Mehrkammerkarpule, insbesondere die 2-Kammerkarpule spielfrei in der Aufnahmevorrichtung. Die zwei Haltearme können vorzugsweise einen Stössel zum Antrieb der beiden Stopfen bilden. Alternative Ausgestaltungen des Antriebsglieds sind ebenfalls möglich. Insbesondere kann das Antriebsglied keine Haltearme aufweisen.

Die Aufnahmevorrichtung kann über eine Gewindeverbindung zwischen der Aufnahmevorrichtung und dem Gehäuse in das Gehäuse eingeführt oder eingeschraubt werden. Durch das Einführen oder Einschrauben der Aufnahmevorrichtung kann ein Stopfen relativ zu der 2-Kammerkarpule innerhalb der 2-Kammerkarpule in die distale Richtung bewegt werden, wobei zunächst die Antriebskraft über ein zwischen den beiden Stopfen vorgesehenes Lösungsmittel auf den anderen Stopfen übertragen werden kann, sodass beide Stopfen distal angetrieben werden können. Sobald der erste Stopfen im Bereich des Bypasses der 2-Kammerkarpule zu liegen kommt, kann der erste Stopfen relativ zu der 2-Kammerkarpule in Ruhe bleiben. Der zweite Stopfen hingegen kann weiter angetrieben werden, sodass das Lösungsmittel über den Bypass gelangen kann, um den in der 2-Kammerkarpule aufgenommenen lyophilisierten Wirkstoff zu lösen. Die Vorrichtung befindet sich in einem abgemischten Zustand, wenn das Lösungsmittel vollständig den Wirkstoff gelöst hat.

Durch das weitere Einführen oder Einschrauben der Aufnahmevorrichtung in das Gehäuse können gleichzeitig beide Stopfen weiter in die distale Richtung verschoben werden. Durch eine Kanüle einer Injektionsnadeleinheit, welche an der Vorrichtung vorgesehenen ist, kann daher überflüssige Luft aus der 2-Kammerkarpule entweichen. Ein gegebenenfalls auftretendes axiales Spiel zwischen der 2-Kammerkarpule und der Aufnahmevorrichtung und dem Gehäuse kann ferner verringert oder sogar beseitigt werden, damit eine sichere und genauere Verabreichung des fluiden Produkts sichergestellt werden kann. Die Vorrichtung befindet sich somit in einem entlüfteten Zustand.

Die Vorrichtung kann ferner ein Blockierelement umfassen. Das Blockierelement ist vorzugsweise relativ zu dem Gehäuse der Vorrichtung drehbar angeordnet. Das Blockierelement kann von einer Blockierposition, in welcher das Betätigungselement an einer axialen Bewegung relativ zu dem Gehäuse gehindert ist, in eine Freigabeposition, in welcher das Betätigungselement relativ zu dem Gehäuse axial bewegbar ist, bewegbar sein. Dazu können die Aufnahmevorrichtung und das Blockierelement jeweils einen Anschlag aufweisen, die zur Bewegung des Blockierelements aus der Blockierposition in die Freigabeposition zusammenwirken. Durch das Einführen oder Einschrauben der Aufnahmevorrichtung in das Gehäuse kann die Aufnahmevorrichtung das Blockierelement mitnehmen. Vorzugsweise kann dadurch das Blockierelement relativ zu dem Gehäuse gedreht werden. Vorzugsweise kann am Ende des abgemischten Zustands der Vorrichtung durch das weitere Einführen oder Einschrauben der Aufnahmevorrichtung in das Gehäuse ein an dem Betätigungselement vorgesehener Vorsprung in die axiale Flucht zu einer an dem Blockierelement vorgesehenen Ausnehmung gelangen. Alternativ kann während dem Abmischen des Lösungsmittels mit dem Wirkstoff das Blockierelement in die Freigabeposition gelangen. In der Freigabeposition des Blockierelements kann das Betätigungselement entlang der Gehäuselangsachse relativ zu dem Gehäuse bewegbar sein. In der Blockierposition kann eine an dem Vorsprung vorgesehene Vorsprungskante des Betätigungselements in Anschlagkontakt mit einer an dem Blockierelement vorgesehenen Blockierelementskante gelangen, um eine relative Bewegung des Betätigungselement entlang der Gehäuselängsachse relativ zu dem Gehäuse zu verhindern. Die Freigabeposition des Blockierelements entspricht vorzugsweise einer Stellung der Mehrkammerkarpule, insbesondere der 2-Kammerkarpule, in der die Mehrkammerkarpule, insbesondere der 2-Kammerkarpule abgemischt oder entlüftet ist.

Das Blockierelement stellt sicher, dass die Vorrichtung erst kurz vor der Anwendung ausgelöst werden kann. Insbesondere bei Mehrkammerkarpulen, insbesondere bei 2-Kammerkarpulen soll eine vorzeitige Auslösung der Vorrichtung verhindert werden. Es soll vor allem sichergestellt werden, dass die Mehrkammerkarpule, insbesondere die 2-Kammerkarpule vollständig abgemischt worden ist oder vollständig abgemischt und entlüftet worden ist, bevor eine Dosis ausgelöst werden kann.

Alternativ können auch andere Elemente in der Vorrichtung vorgesehen sein, welche ein unbeabsichtigtes oder vorzeitiges Auslösen der Vorrichtung verhindern.

Ferner kann eine entsprechende Anzeigevorrichtung, in Form einer Ziffer, eines Buchstaben oder eines anderen Zeichens an der Aufnahmevorrichtung vorgesehen sein, wobei durch ein an dem Gehäuse vorgesehenes Fenster der Ausgangszustand und/oder der abgemischte Zustand und/oder der entlüftete Zustand der Vorrichtung anzeigt werden kann.

Ferner kann die Aufnahmevorrichtung ein oder mehrere Rastarme aufweisen, welche in Eingriff mit einer oder mehreren in dem Gehäuse vorgesehenen Rastöffnungen oder Rastlängsnuten rasten können, um eine Rückschiebe- oder Rückdrehsicherung auszubilden, sodass eine sichere Verabreichung des fluiden Produkts sichergestellt werden kann. Die Rückschiebe- oder Rückdrehsicherung kann ferner ein akustisches Signal erzeugen, um anzuzeigen, in welchem Zustand sich die Vorrichtung befindet.

Ferner kann an dem Antriebsglied ein Arm vorgesehen sein, welcher in einem verriegelten Zustand der Vorrichtung in eine Aussparung der Aufnahmevorrichtung einrastbar ist. Dieses Einrasten kann ferner ein akustisches Geräusch erzeugen, um anzuzeigen, dass die Vorrichtung in dem verriegelten oder benutzen Zustand ist. Die Vorrichtung kann vorzugsweise zudem aufgrund des Eingriffs zwischen dem Arm des Antriebsglieds und der Aussparung der Aufnahmevorrichtung nicht mehr verwendet werden.

Um eine erfindungsgemässe Vorrichtung zu montieren kann ein Halteelement mit einem Eingriffselement in proximaler Richtung ein Gehäuse eingefügt werden. Ein Blockierelement kann mittels einer oder mehreren Führungen in distaler Richtung in das Gehäuse geführt werden. Ein mit einer Feder aufgenommenes Antriebsglied, welches ein Halteelement aufweist, kann danach in proximaler Richtung in das Gehäuse eingeführt und relativ zu dem Gehäuse gedreht oder geschraubt werden, wobei ein an dem Halteelement vorgesehenes Element zur Führung der Feder dient. Danach kann ein Betätigungselement in distaler Richtung in das Gehäuse eingesetzt werden. Eine Aufnahmevorrichtung, welche eine Karpule, insbesondere eine Mehrkammerkarpule aufgenommen hat, kann in proximaler Richtung in das Gehäuse geschraubt werden, derart, dass die Vorrichtung in einem Ausgangszustand ist.

Die Erfindung wird im Folgenden anhand mehrerer Figuren beschrieben. Die hierbei offenbarten Merkmale bilden die Erfindung vorteilhaft weiter. Es zeigen:
- Figur 1: eine Explosionsansicht einer ersten Ausführungsform einer erfindungsgemässen Vorrichtung.
- Figur 2a: eine Aussenansicht der Vorrichtung aus Figur 1 in einem Ausgangszustand, in welchem die Vorrichtung ausgeliefert wird.
- Figur 2b: eine Längsschnittansicht der Vorrichtung gemäss Figur 2a, wobei die Längsschnittansicht der in der Figur 2a eingezeichneten Schnittlinie A-A entspricht.
- Figur 2c: eine Längsschnittansicht der Vorrichtung aus Figur 1, wobei die Ansicht gegenüber der Figur 2a um 90 Grad gedreht ist.
- Figur 2d: eine Detailansicht der Vorrichtung aus Figur 1, wobei das Blockierelement und das Betätigungselement und dessen Zusammenwirken in dem Ausgangszustand ersichtlich ist.
- Figur 3a: eine Aussenansicht der Vorrichtung aus Figur 1 in einem abgemischten Zustand.
- Figur 3b: eine Längsschnittansicht der Vorrichtung gemäss Figur 3a, wobei die Längsschnittansicht der in der Figur 3a eingezeichneten Schnittlinie A-A entspricht.
- Figur 3c: eine Längsschnittansicht der Vorrichtung aus Figur 1, wobei die Ansicht gegenüber der Figur 3a um 90 Grad gedreht ist.
- Figur 3d: eine Detailansicht der Vorrichtung aus Figur 1, wobei die Aufnahmevorrichtung, das Blockierelement und das Betätigungselement und dessen Zusammenwirken in dem abgemischten Zustand ersichtlich ist.
- Figur 4a: eine Aussenansicht der Vorrichtung aus Figur 1 in einem entlüfteten Zustand.
- Figur 4b: eine Längsschnittansicht der Vorrichtung gemäss Figur 4a, wobei die Längsschnittansicht der in der Figur 4a eingezeichneten Schnittlinie A-A entspricht.
- Figur 4c: eine Längsschnittansicht der Vorrichtung aus Figur 1, wobei die Ansicht gegenüber der Figur 4a um 90 Grad gedreht ist.
- Figur 4d: eine Detailansicht der Vorrichtung aus Figur 1, wobei die Aufnahmevorrichtung, das Blockierelement und das Betätigungselement und dessen Zusammenwirken in dem entlüfteten Zustand ersichtlich ist.
- Figur 5a: eine Aussenansicht der Vorrichtung aus Figur 1 in einem ausgelösten Zustand, wobei eine Abgabe oder Ausschüttung einer Dosis aus der Vorrichtung ausgelöst ist.
- Figur 5b: eine Längsschnittansicht der Vorrichtung gemäss Figur 5a, wobei die Längsschnittansicht der in der Figur 5a eingezeichneten Schnittlinie A-A entspricht.
- Figur 5c: eine Längsschnittansicht der Vorrichtung aus Figur 1, wobei die Ansicht gegenüber der Figur 5a um 90 Grad gedreht ist.
- Figur 5d: eine Detailansicht der Vorrichtung aus Figur 1, wobei das Blockierelement und das Betätigungselement und dessen Zusammenwirken in dem ausgelösten Zustand ersichtlich ist.
- Figur 6a: eine Aussenansicht der Vorrichtung aus Figur 1 in einem ausgeschütteten Zustand.
- Figur 6b: eine Längsschnittansicht der Vorrichtung gemäss Figur 5a, wobei die Längsschnittansicht der in der Figur 6a eingezeichneten Schnittlinie A-A entspricht.
- Figur 6c: eine Längsschnittansicht der Vorrichtung aus Figur 1, wobei die Ansicht gegenüber der Figur 5a um 90 Grad gedreht ist.

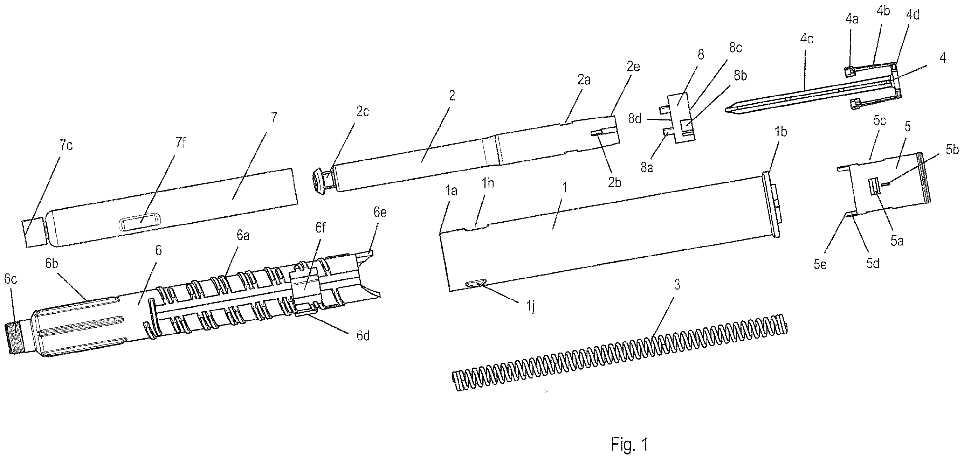

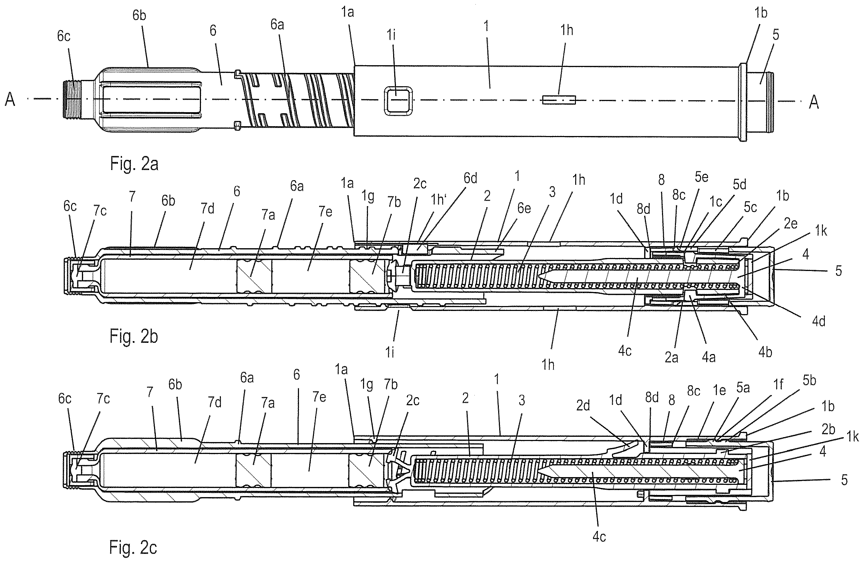

In der Figur 1 ist eine Explosionsansicht einer ersten Ausführungsform der erfindungsgemässen Vorrichtung dargestellt. Die Vorrichtung umfasst ein Gehäuse (1) mit einem distalen (1a) und einem proximalen Ende (1b) oder Abschnitt. Das Gehäuse (1) ist hülsenförmig ausgebildet. Das proximale Ende (1b) oder Abschnitt des Gehäuses umfasst ferner einen proximalen Gehäuseanschlag (1k; Figur 2b). Der proximale Gehäuseanschlag (1k; Figur 2b) ist an einer Mantelinnfläche des proximalen Ende (1b) oder Abschnitt des Gehäuses (1) vorgesehen. An einer Mantelaussenfläche des Gehäuses (1) kann ein ober mehrere Nocken (1j) vorgesehen sein, welche dazu dienen, ein Rollen der Vorrichtung beispielsweise auf einem Tisch zu verhindern. Ferner umfasst die Vorrichtung ein Antriebsglied (2), wobei das Antriebsglied (2) axial bewegbar in dem Gehäuse (1) gelagert ist. Das Antriebsglied (2) ist hülsenförmig ausgebildet. Der Aussendurchmesser des Antriebsglieds (2) kann vorzugsweise am distalen Ende und am proximalen Ende unterschiedlich sein, wobei der Innendurchmesser des Antriebsglieds am distalen und am proximalen Ende des Antriebsglieds gleich ist. Die Grösse des Innendurchmessers des Antriebsglieds (2) ist derart ausgelegt, dass eine vorgespannte Feder (3) zumindest teilweise von dem Antriebsglieds (2) aufgenommen ist. Der Aussendurchmesser des Antriebsglieds (2) ist besonders bevorzugt am proximalen Ende grösser als am distalen Ende. Des Weiteren umfasst die Vorrichtung ein Halteelement (4). Das Halteelement (4) dient zum Halten des mit einer Kraft der Feder beaufschlagten Antriebglieds (2). Die vorgespannte Feder (3) ist an einem distalen Ende an dem Antriebsglied (2) und an einem proximalen Ende an dem Halteelement (4) abgestützt. An dem Halteelement (4) ist ein Eingriffselement (4a) vorgesehen, welches vorzugsweise als Nocke (4a) ausgebildet ist. Das Halteelement (4) weist einen spannbaren Arm (4b), insbesondere einen radial nach aussen elastisch auslenkbaren beziehungsweise einen radial nach innen elastisch einlenkbaren Arm (4b) auf, an welchem das Eingriffselement (4a), insbesondere die Nocke (4a) angeordnet ist. Besonders bevorzugt weist das Haltelement (4) zwei oder mehrere Eingriffselemente (4a), insbesondere zwei oder mehrere Nocken (4a) auf, welche an entsprechenden Armen (4b) des Halteelements (4) angeordnet sind. Besonders bevorzugt umfasst das Halteelement (4) einen oder mehrere Stege (4d), welche jeweils zwei Arme (4b) des Halteelements (4) miteinander verbindet. An dem Antriebsglied (2) ist ein Eingriffselement (2a) vorgesehen, welches vorzugsweise als Aussparung (2a) ausgebildet ist. Besonders bevorzugt weist das Antriebsglied (2) zwei oder mehrere Eingriffselemente (2a), insbesondere zwei oder mehrere Aussparungen (2a) auf. Das Eingriffselement (4a) des Halteelements (4) und das Eingriffselement (2a) des Antriebsglieds (2) sind derart ausgebildet, dass in einem Ausgangszustand der Vorrichtung das Antriebsglied (2) durch einen Eingriff zwischen dem Eingriffselement (4a) des Halteelements (4) und dem Eingriffselement (2a) des Antriebsglieds (2) in einer proximalen Position haltbar ist. Ferner sind das Eingriffselement (4a) des Halteelements (4) und das Eingriffselement (2a) des Antriebselements (2) derart ausgebildet, dass in einem ausgelösten Zustand der Vorrichtung das Eingriffselement (4a) des Halteelements (4) ausser Eingriff mit dem Eingriffselement (2a) des Antriebsglieds (2) sein kann. Das Halteelement (4) ist zumindest in dem Ausgangszustand in proximaler Richtung von dem proximalen Ende (1b) oder Abschnitt des Gehäuses (1), insbesondere von dem proximalen Gehäuseanschlag (1k; Figur 2b) beabstandet. Das Halteelement (4) und das Antriebselement (2) können relativ zueinander drehfest gelagert sein. Dazu kann der Steg (4d) des Halteelements (4) zumindest in dem Ausgangszustand der Vorrichtung in einer an dem proximalen Ende des Antriebglieds (2) vorgesehenen Kerbe (2e) angeordnet sein. Um das Halteelement (4) und das Gehäuse (1) relativ zueinander drehfest zu lagern, kann der Arm (4b) des Halteelements (4) in einer an dem Gehäuse (1) vorgesehenen Längsaussparung (nicht ersichtlich) oder Längsnut (nicht ersichtlich) aufgenommen sein. An dem Gehäuse (1) ist ferner ein Ausschüttungsanschlag (1d; Figur 2c) vorgesehen, welcher in Anschlagkontakt mit einem an dem Antriebsglied (2) angebrachten Ausschüttungsanschlag (2b) gelangbar ist, um eine Dosis zu verabreichen. Alternativ können auch mehrere Ausschüttungsanschläge (1d; Figur 2c) des Gehäuses (1) und mehrere Ausschüttungsanschläge (2b) des Antriebsglied (2) vorgesehen sein. Der Ausschüttungsanschlag (2b) des Antriebsglieds (2) ist axial in die proximale Richtung versetzt zu dem Eingriffselement (2a) des Antriebsglieds (2) an dem Antriebsglieds (2) angeordnet. Ferner ist der Ausschüttungsanschlag (2b) des Antriebsglieds (2) in Umfangsrichtung um 90 Grad versetzt zu dem Eingriffselement (2a) des Antriebsglieds (2) an dem Antriebsglied (2) angeordnet. Des Weiteren kann der Ausschüttungsanschlag (2b) des Antriebsglieds in einer an dem Gehäuse (1) vorgesehenen Längsaussparung (nicht ersichtlich) oder Längsnut (nicht ersichtlich) aufgenommen sein, um das Antriebsglied (2) und das Gehäuse (1) relativ zueinander drehfest zu lagern. Ferner umfasst das Halteelement (4) ein Element (4c) zur Führung der Feder (3). Das Element (4c) des Halteelements (4) kann dornförmig ausgebildet sein. Die Feder (3) kann vorzugsweise als Wendelfeder (3) ausgebildet sein. Die wendelförmige Feder (3) umgibt das Element (4c) des Halteelements (4) derart, dass die Feder (3) durch das Element (4c) des Halteelements (4) führbar ist. Die Vorrichtung umfasst ferner ein Betätigungselement (5) zum Auslösen der Vorrichtung. Das Betätigungselement (5) ist relativ zu dem Gehäuse (1) axial bewegbar. Das Betätigungselement (5) ist drehfest zu dem Gehäuse (1) angeordnet. Dazu weist das Betätigungselement (5) an einer Mantelaussenfläche eine erste Nocke (5a) auf, welche in eine an der Mantelinnenfläche des Gehäuses (1) vorgesehene Längsnut (1e; Figur 2c) ragt. An dem Betätigungselement (5) ist ferner eine zweite Nocke (5b) vorgesehen, welche in proximaler Richtung versetzt zu der ersten Nocke (5a) an dem Betätigungselement (5) angeordnet ist, welche ebenfalls in die Längsnut (1e; Figur 2c1) des Gehäuses (1) ragt. Die Längsnut (1e) des Gehäuses (1) ist durch eine an der Mantelinnenfläche des Gehäuses angeordnete Rippe (1f) in einen distalen und einen proximalen Abschnitt unterteilt. Die erste (5a) und die zweite Nocke (5b) des Betätigungselements (5) und die Rippe (1f) des Gehäuses (1) sind derart ausgebildet und wirken derart zusammen, dass eine relative Bewegung des Betätigungselements (5) zu dem Gehäuse (1) in die proximale Richtung verhinderbar ist, um eine fehlerhafte Handhabung zu verhindern. Des Weiteren sind die erste (5a) und die zweite Nocke (5b) des Betätigungselements (5) und die Rippe (1f) des Gehäuses (1) derart ausgebildet und wirken derart zusammen, dass eine relative Bewegung des Betätigungselements (5) zu dem Gehäuse (1) in die distale Richtung möglich ist, um eine Dosis aus der Vorrichtung auszuschütten. Ferner weist das Betätigungselement (5) eine Ausnehmung (5c) auf, welche derart ausgebildet ist, dass das Eingriffselement (4a), insbesondere die Nocke (4a) des Halteelements (4) in die Ausnehmung (5c) des Betätigungselements (5) ragbar ist. An dem Gehäuse (1) kann vorzugsweise ferner ein Vorsprung (1c; Figur 2b) vorgesehen sein, der dazu dient, eine relative Bewegung des Halteelements (4) zu dem Gehäuse (1) in die distale Richtung zu begrenzen. Die Vorrichtung umfasst ferner eine Aufnahmevorrichtung (6) zur Aufnahme des fluiden Produkts. Die Aufnahmevorrichtung (6) ist relativ zu dem Gehäuse (1) drehbar gelagert. An einer Mantelaussenseite der Aufnahmevorrichtung (6) ist ein Aussengewinde (6a) vorgesehen, welche in einem Gewindeeingriff mit einem an der Mantelinnenfläche des Gehäuses (1) angebrachtes Innengewinde (1g; Figur 2a) ist. An der Mantelaussenfläche der Aufnahmevorrichtung (6) können vorzugsweise eine oder mehrere Längsrippen (6b) vorgesehen sein, damit der Benutzer mit der Hilfe der Längsrippen (6b) der Aufnahmevorrichtung (6) die Aufnahmevorrichtung in das Gehäuse (1) einführen oder einschrauben kann. Die Aufnahmevorrichtung (6) kann vorzugsweise einen oder mehrere Rastarme (6d) aufweisen, welche in Eingriff mit einer oder mehreren in dem Gehäuse vorgesehenen Rastöffnungen (1h) rasten können, um sicherzustellen, dass die Aufnahmevorrichtung (6) nur in die distale Richtung in das Gehäuse (1) eingeführt oder eingeschraubt werden kann. Dadurch kann eine Rückschiebe- oder Rückdrehsicherung ausgebildet sein, sodass eine sichere Verabreichung des fluiden Produkts sichergestellt werden kann. Ferner kann an der Aufnahmevorrichtung (6) eine Anzeigevorrichtung (nicht ersichtlich) in Form von Ziffern, Buchstaben oder anderen Zeichen angeordnet sein, welche durch ein oder mehrere an dem Gehäuse vorgesehene Fenster (1i) ersichtlich sind. Dadurch kann angezeigt werden, in welchem Zustand sich die Vorrichtung befindet. In der Aufnahmevorrichtung (6) ist eine Mehrkammerkarpule (7), insbesondere eine 2-Kammerkarpule (7) aufgenommen. Ferner kann die Rückschiebe- oder Rückdrehsicherung ein akustisches Signal erzeugen, um anzuzeigen, in welchem Zustand sich die Vorrichtung befindet. Das Antriebsglied (2) umfasst ferner einen spannbaren Arm (2d), insbesondere einen radial nach aussen elastisch auslenkbaren beziehungsweise einen radial nach innen elastisch einlenkbaren Arm (2d) auf, welcher in eine an der Aufnahmevorrichtung (6) vorgesehene Aussparung (6f) einrasten kann. Ferner kann der Arm (2b) des Antriebsglieds (2) beim Einrasten in die Aussparung (6f) der Aufnahmevorrichtung (6) ein akustisches Signal erzeugen, durch welches angezeigt werden kann, dass die Ausschüttung des fluiden Produkts beendet ist. An dem distalen Ende des Antriebsglieds (2) sind ein oder mehrere, besonders bevorzugt zwei Haltearme (2c) angeordnet, welche zumindest in dem Ausgangszustand der Vorrichtung die 2-Kammerkarpule (7) in die distale Richtung drücken. Die 2-Kammerkarpule (7) wird mittels Haltearme (2c) des Antriebsglieds (2) in einer definierten Position relativ zu der Aufnahmevorrichtung (6) gehalten. Eine axiale Bewegung der 2-Kammerkarpule (7) in der Aufnahmevorrichtung (6) wird dadurch verhindert. Die 2-Kammerkarpule (7) weist einen ersten Stopfen (7a) und einen zweiten Stopfen (7b) auf. Der zweite Stopfen (7b) schliesst die 2-Kammer-Karpule (7) am proximalen Ende ab. Am distalen Ende der 2-Kammerkarpule (7) ist eine Membran (7c) vorgesehen. Die Membran (7c) kann durch eine Kanüle (nicht ersichtlich) einer Injektionsnadeleinheit (nicht ersichtlich) durchstochen werden. Zur Verbindung der Injektionsnadeleinheit (nicht ersichtlich) mit der Vorrichtung ist an dem distalen Ende der Aufnahmevorrichtung (6) ein Injektionsnadeleinheitsverbindungselement (6c) in Form eines Aussengewindes (6c) vorgesehen, welches in einem Gewindeeingriff mit einem an der Injektionsnadeleinheit (nicht ersichtlich) angeordneten Innengewinde (nicht ersichtlich) sein kann. Alternativ können andere Verbindungen zwischen der Aufnahmevorrichtung (6) und der Injektionsnadeleinheit (nicht ersichtlich) vorgesehen sein, beispielsweise eine Schnappverbindung oder eine Rastverbindung. Zwischen der Membran (7c) und dem ersten Stopfen (7a) der 2-Kammerkarpule (7) ist eine erste Kammer (7d) ausgebildet, in welcher ein lyophilisierter Wirkstoff aufgenommen ist. Zwischen dem ersten Stopfen (7a) und dem zweiten Stopfen (7b) wird eine zweite Kammer (7e) der 2-Kammerkarpule (7) gebildet, in welcher das Lösungsmittel für den Wirkstoff gelagert ist. Die 2-Kammerkarpule (7) umfasst ferner einen Bypass (7f), wobei das Lösungsmittel über den Bypass (7f) in die erste Kammer (7d) gelangen kann, um den Wirkstoff mit dem Lösungsmittel abzumischen. Die Vorrichtung umfasst ferner ein Blockierelement (8). Das Blockierelement (8) ist hülsenförmig ausgebildet. Das Blockierelement (8) ist relativ zu dem Gehäuse (1) drehbar und axialfest angeordnet. Eine an dem Blockierelement (8) vorgesehene Blockierelementauflage (8d) ist an dem Gehäuse (1) abgestützt. Um das Blockierelement (8) relativ zu dem Gehäuse (1) radial zu positionieren, kann eine Nut-/Nockenverbindung (nicht ersichtlich) vorzugsweise zwischen der Mantelaussenfläche des Blockierelements (8) und der Mantelinnenfläche des Gehäuses (1) vorgesehen sein. Das Blockierelement (8) ist zum Blockieren des Betätigungselements (5) vorgesehen, wobei durch Drehung der Aufnahmevorrichtung (6) relativ zum Gehäuse (1) das Blockierelement (8) von einer Blockierposition in eine Freigabeposition bewegbar ist. Die Aufnahmevorrichtung (6) weist einen oder mehrere Anschläge (6e) auf, welche mit einem oder mehreren an dem Blockierelement (6) vorgesehenen Anschläge (8a) in Anschlagkontakt gelangen können. Der Anschlag (8a) des Blockierelements (8) erstreckt sich von der Blockierelementauflage (8d) des Blockierelements (8) in die distale Richtung. Der Anschlag (6e) der Aufnahmevorrichtung (6) ragt von dem proximalen Ende der Aufnahmevorrichtung (6) in die proximale Richtung ab. Der Anschlag (6e) der Aufnahmevorrichtung (6) kann derart mit dem Anschlag (8a) des Blockierelements (8) zusammenwirken, dass das Blockierelement (8) aus der Blockierposition in die Freigabeposition gelangen kann. Das Betätigungselement (5) und das Blockierelement (8) sind derart ausgebildet, dass das Betätigungselement (5) entlang einer Gehäuselängsachse relativ zum Gehäuse (1) bewegbar sein kann oder relativ zum Gehäuse (1) axial fest sein kann. Das Betätigungselement (5) kann einen oder mehrere Vorsprünge (5d) aufweisen, welche in eine oder in mehrere Ausnehmungen (8b) des Blockierelements (8) aufnehmbar sind, wobei das Betätigungselement (5) entlang der Gehäuselängsachse des Gehäuses (1) bewegbar ist. Der Vorsprung (5d) des Betätigungselements (5) ist an einem distalen Ende des Betätigungselements (5) angeordnet und erstreckt sich in die distale Richtung. Die Ausnehmung (8b) ist an einem proximalen Ende des Blockierelements (8) angeordnet und erstreckt sich in die distale Richtung. Ferner kann an dem Betätigungselement (5) eine Vorsprungskante (5e), insbesondere an dem Vorsprung (5d) des Betätigungselements (5) eine Vorsprungskante (5e) vorgesehen sein, welche mit einer an dem proximalen Ende des Blockierelements (8) angeordneten Blockierelementkante (8c) in Anschlagkontakt gelangen kann, um eine Bewegung des Betätigungselements (5) entlang der Gehäuselängsachse relativ zum Gehäuse (1) zu verhindern.

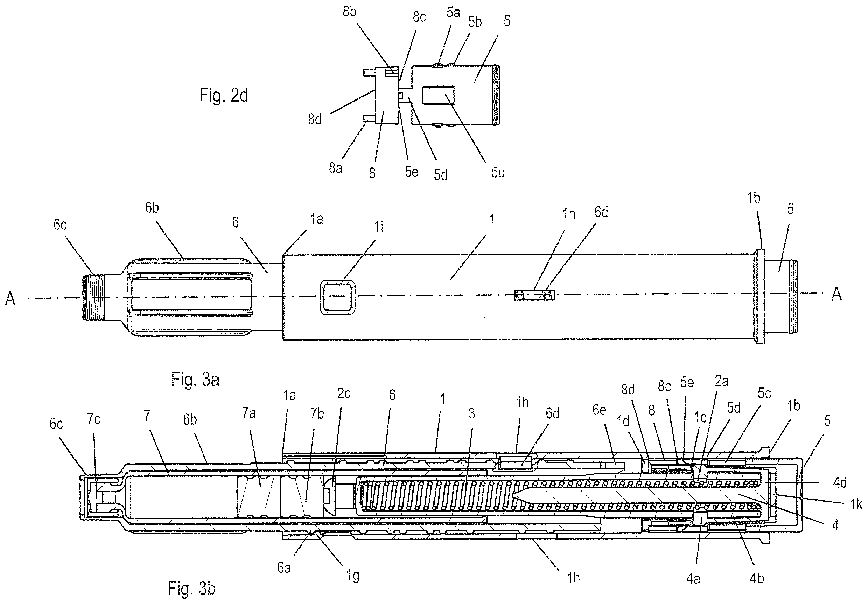

In der Figur 2b ist eine Längsschnittansicht der Vorrichtung aus der Figur 1 in einem Ausgangszustand, in welchem die Vorrichtung ausgeliefert wird, dargestellt, wobei die Längsschnittansicht der in der Figur 2a eingezeichneten Schnittlinie A-A entspricht. Ferner ist in der Figur 2c eine Längsschnittansicht der Vorrichtung gemäss Figur 2b gezeigt, wobei die Längsschnittansicht gegenüber der Figur 2a um 90 Grad gedreht ist. Das Eingriffselement (4a), insbesondere die Nocke (4a) des Halteelements (4) ist in Eingriff mit dem Eingriffselement (2a), insbesondere mit der Aussparung (2a) des Antriebsglieds (2). Das Betätigungselement (5) ist relativ zu dem Halteelement (4) derart angeordnet, dass das Eingriffselement (4a), insbesondere die Nocke (4a) des Halteelements (4) in Eingriff mit dem Eingriffselements (2a), insbesondere mit der Aussparung (2a) des Antriebsglieds gehalten werden kann. Die Feder (3) ist am distalen Ende an dem Antriebsglied (2) und an dem proximalen Ende an dem Halteelement (4) vorgespannt abgestützt. Ferner ist das Betätigungselement (5) derart relativ zu dem Gehäuse (1) angeordnet, dass zwischen der ersten (5a) und der zweiten (5b) Nocke des Betätigungselements (5) die Rippe (1f) des Gehäuses (1) angeordnet ist. Der Arm (4b) des Halteelements (4) wird mittels einer Mantelinnenfläche des Betätigungselements (5) radial nach innen elastisch vorgespannt gehalten. Das Antriebsglied (2) wird mit einer in die distale Richtung wirkenden Kraft der Feder (3) beaufschlagt. Das Halteelement (4) ist in proximaler Richtung von dem proximalen Gehäuseanschlag (1k) beabstandet. Das Eingriffselement (4a), insbesondere die Nocke (4a) des Halteelements (4) ist in proximaler Richtung an dem Antriebsglied (2), insbesondere an dem proximalen Ende des Eingriffselements (2a), insbesondere an der Aussparung (2a) des Antriebsglieds (2) abgestützt. Das proximale Ende des Antriebsglieds (2) ist in Anschlagkontakt mit dem Gehäuse (1). Das distale Ende des Antriebsglieds (2) ist in Anschlagkontakt mit der in der Aufnahmevorrichtung (6) aufgenommenen 2-Kammerkarpule (7). Der Steg (4d) des Halteelements (4) ist in der Kerbe (2e) des Antriebsglieds (2) angeordnet. Der Arm (2d) des Halteelements (2) ist durch die Mantelinnenfläche des Gehäuses (1) radial nach innen vorgespannt. Die zwei Haltearme (2c) des Antriebsglieds (2) stossen die 2-Kammerkarpule (7) in die distale Richtung. Die 2-Kammerkarpule (7) wird mittels Haltearme (2c) des Antriebsglieds (2) in einer definierten Position relativ zu der Aufnahmevorrichtung (6) gehalten. Eine axiale Bewegung der 2-Kammerkarpule (7) in der Aufnahmevorrichtung (6) wird dadurch verhindert. Die zwei Haltearme (2c) des Antriebsglieds (2) ragen seitlich von der Längsachse des Antriebsglieds (2) ab. Die zwei Haltearme (2c) des Antriebsglieds (2) sind zueinander aufgespreizt und halten die 2-Kammerkarpule (7) spielfrei in der Aufnahmevorrichtung (6). Dazu sind die an den Haltearmen (2c) des Antriebsglieds (2) vorgesehenen schrägen Flächen in Anschlagkontakt mit einer proximalen Kante der 2-Kammerkarpule (7). Die Aufnahmevorrichtung (6) ist über die Gewindeverbindung zwischen dem Aussengewinde (6a) der Aufnahmevorrichtung (6) und dem Innengewinde (1g) des Gehäuses (1) in Eingriff mit dem Gehäuse (1). Die Anzeigevorrichtung (nicht ersichtlich), insbesondere eine entsprechende Ziffer, Zeichen oder Buchstabe ist derart durch das an dem Gehäuse (1) vorgesehene Fenster (1i) ersichtlich, dass angezeigt werden kann, dass sich die Vorrichtung in dem Ausgangszustand befindet. Ferner ist der Rastarm (6d) der Aufnahmevorrichtung (6) in einer Rastöffnung (1h) des Gehäuses oder alternativ in einer Rastlängsnut (1h') des Gehäuses (1), insbesondere in einer am distalen Ende der Vorrichtung angeordneten Rastöffnung (1h) oder Rastlängsnut (1h'), welche an der Mantelinnenfläche des Gehäuses (1) angeordnet ist, eingerastet. Die Blockierelementauflage (8d) des Blockierelements (8) ist an dem Gehäuse (1) abgestützt. Ferner ist das Blockierelement (8) radial relativ zu dem Gehäuse (1) über eine Nut-/Nockenverbindung (nicht ersichtlich) positioniert. Das Blockierelement (8) befindet sich in der Blockierposition, in der eine Betätigung des Betätigungselements (5) blockiert ist. Das Betätigungselement (5) kann nicht entlang der Gehäuseachse relativ zum Gehäuse bewegt werden. Wie in der Figur 2d ersichtlich ist, gelangt dabei die Vorsprungskante (5e) des Vorsprungs (5d) des Betätigungselements (5) in Anschlagkontakt mit der Blockierelementskante (8c) des Blockierelements (8).

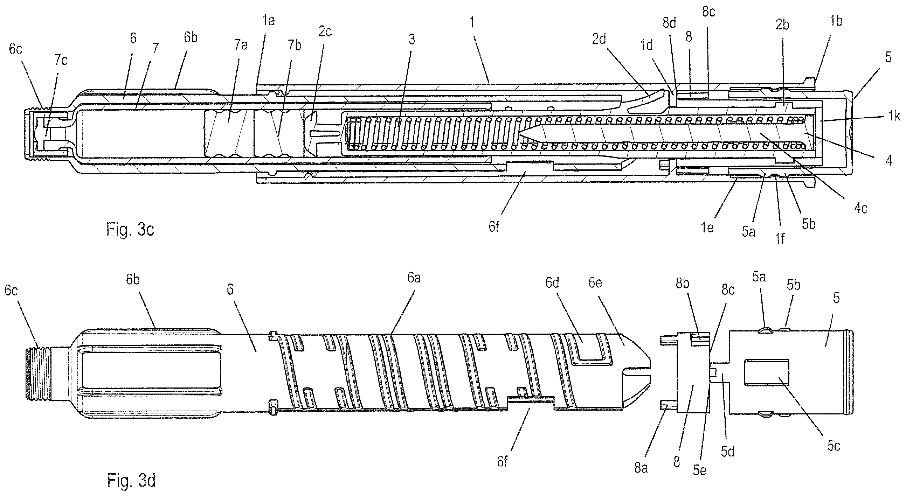

In der Figur 3b ist eine Längsschnittansicht der Vorrichtung aus der Figur 1 in einem abgemischten Zustand dargestellt, wobei die Längsschnittansicht der in der Figur 3a eingezeichneten Schnittlinie A-A entspricht. Ferner ist in der Figur 3c eine Längsschnittansicht der Vorrichtung gemäss Figur 3b gezeigt, wobei die Längsschnittansicht gegenüber der Figur 3a um 90 Grad gedreht ist. Auf das Injektionsnadeleinheitsverbindungselement (6c) der Aufnahmevorrichtung (6) kann eine Injektionsnadeleinheit (nicht ersichtlich) aufgesetzt werden. Eine Kanüle (nicht ersichtlich) der Injektionsnadeleinheit (nicht ersichtlich) kann die Membran (7c) der 2-Kammerkarpule (7) durchstechen. Aus dem Ausgangszustand gemäss Figur 2b und Figur 2c wird die mit der 2-Kammerkarpule (7) aufgenommene Aufnahmevorrichtung (6) relativ zu dem Gehäuse (1) in proximaler Richtung in das Innere des Gehäuses (1) eingeführt oder eingeschraubt. Dazu wird die Aufnahmevorrichtung (6) über die Gewindeverbindung zwischen dem Aussengewinde (6a) der Aufnahmevorrichtung (6) und dem Innengewinde (1g) des Gehäuses (1) in das Gehäuse (1) eingeführt oder eingeschraubt. Durch das Einführen oder Einschrauben der Aufnahmevorrichtung (6) rutschen die schrägen Flächen der Haltearme (2c) des Antriebsglieds (2) an der proximalen Kante der 2-Kammerkarpule (7) ab, wobei die Haltearme (2c) des Antriebsglieds radial nach innen in Richtung der Längsachse des Antriebsglieds (2) bewegt werden. Die Haltearme (2c) des Antriebsglieds (2) bewegen sich dabei nach innen aufeinander zu und bilden einen Stössel für den zweiten Stopfen (7b) der 2-Kammerkarpule (7). Das Halteelement (4) ist in proximaler Richtung von dem proximalen Gehäuseanschlag (1k) beabstandet. Das Eingriffselement (4a), insbesondere die Nocke (4a) des Halteelements (4) ist in proximaler Richtung an dem Antriebsglied (2), insbesondere an dem proximalen Ende des Eingriffselements (2a), insbesondere der Aussparung (2a) des Antriebsglieds (2) abgestützt. Das proximale Ende des Antriebsglieds (2) ist in Anschlagkontakt mit dem Gehäuse (1). Der Steg (4d) des Halteelements (4) ist in der Kerbe (2e) des Antriebsglieds (2) angeordnet. Durch das Einführen oder Einschrauben der Aufnahmevorrichtung (6) in das Gehäuse (1) wird ferner der zweite Stopfen (7b) der 2-Kammerkarpule (7) innerhalb der 2-Kammerkaprule (7) in die distale Richtung bewegt. Dadurch wird zunächst die Antriebskraft durch das Lösungsmittel in der zweiten Kammer (7e) auf den ersten Stopfen (7a) übertragen, sodass beide Stopfen (7a, 7b) distal angetrieben werden. Sobald der erste Stopfen (7a) im Bereich des Bypasses (7f) der 2-Kammerkarpuel (7) zu liegen kommt, bleibt der erste Stopfen (7a) relativ zu der 2-Kammerkarpule (7) in Ruhe. Der zweite Stopfen (7b) hingegen wird weiter angetrieben, sodass das Lösungsmittel aus der zweiten Kammer (7e) über den Bypass (7f) in die erste Kammer (7d) gelangt und den dort befindlichen Wirkstoff lösen kann. Der zweite Stopfen (7b) wird solange angetrieben, bis er auf den ersten Stopfen (7a) trifft. Dadurch wird der abgemischte Zustand der Vorrichtung erreicht. Während diesem Vorgang wird die Aufnahmevorrichtung (6) fortwährend weiter in das Gehäuse (1) eingeführt oder eingeschraubt. Die Anzeigevorrichtung (nicht ersichtlich), insbesondere eine entsprechende Ziffer, ein entsprechender Buchstabe oder ein anderes entsprechendes Zeichen der Aufnahmevorrichtung (6) ist derart durch das an dem Gehäuse (1) vorgesehene Fenster (1i) ersichtlich, dass angezeigt wird, dass sich die Vorrichtung in dem abgemischten Zustand befindet. Ferner ist der Rastarm (6d) der Aufnahmevorrichtung (6) in einer Rastöffnung (1h) des Gehäuses (1) insbesondere in einer zwischen dem distalen Ende und proximalen Ende der Vorrichtung angeordneten Rastöffnung (1h) des Gehäuses (1) eingerastet.

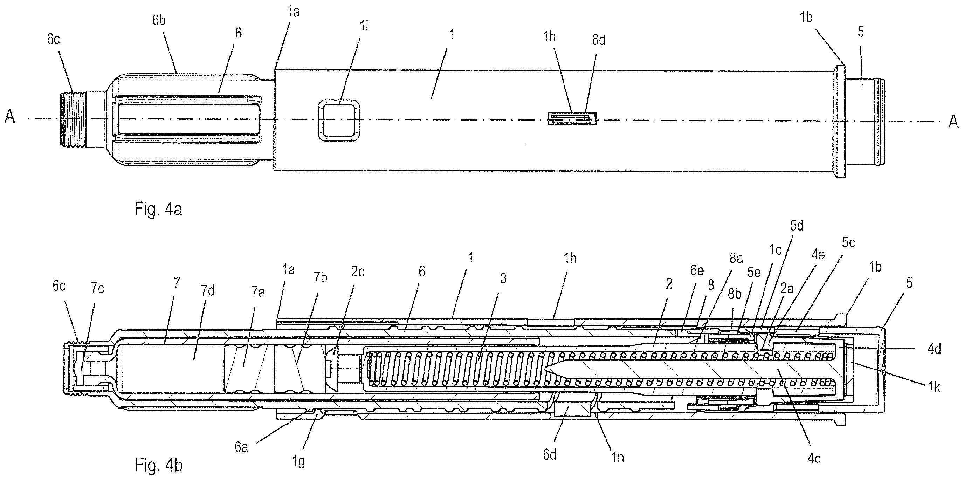

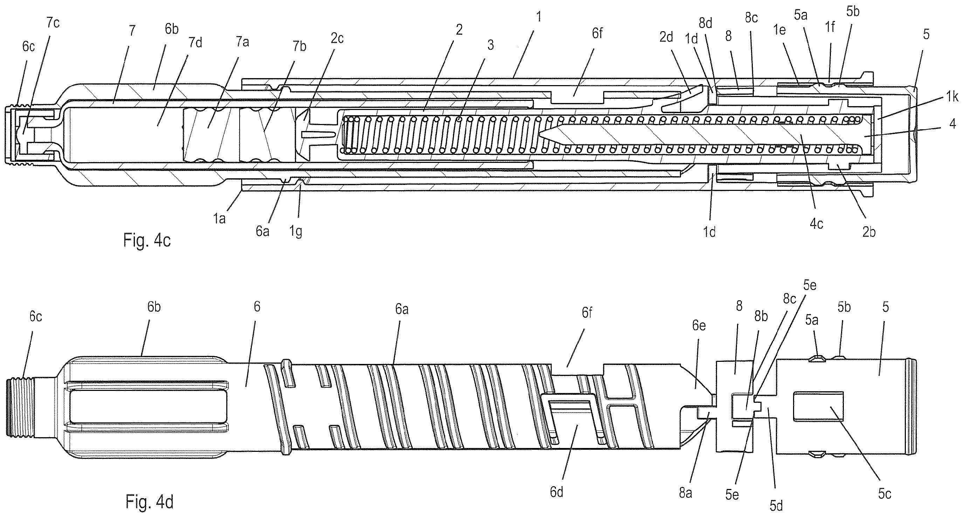

In der Figur 4b ist eine Längsschnittansicht der Vorrichtung aus der Figur 1 in einem entlüfteten Zustand dargestellt, wobei die Längsschnittansicht der in der Figur 4a eingezeichneten Schnittlinie A-A entspricht. Ferner ist in der Figur 4c eine Längsschnittansicht der Vorrichtung gemäss Figur 4b gezeigt, wobei die Längsschnittansicht gegenüber der Figur 4a um 90 Grad gedreht ist. Aus dem abgemischten Zustand gemäss Figur 3a und Figur 3b wird die Aufnahmevorrichtung (6) weiter in die proximale Richtung in das Gehäuse (1) eingeführt oder eingeschraubt. Während einem weiteren Vorschub der Aufnahmevorrichtung (6) gegenüber dem Gehäuse (1) werden gleichzeitig beide Stopfen (7a, 7b) weiter in die distale Richtung verschoben. Durch die Kanüle (nicht ersichtlich) der Injektionsnadeleinheit (nicht ersichtlich) kann daher überflüssige Luft aus der ersten Kammer (7d) der 2-Kammerkarpule (7) entweichen. Zudem kann ein gegebenenfalls auftretendes axiales Spiel zwischen der 2-Kammerkarpule (7) und der Aufnahmevorrichtung (6) und dem Gehäuse (1) verringert oder sogar beseitigt werden, damit eine sichere und genauere Verabreichung des fluiden Produkts sichergestellt werden kann. Das Halteelement (4) ist in proximaler Richtung von dem proximalen Gehäuseanschlag (1k) beabstandet. Das Eingriffselement (4a), insbesondere die Nocke (4a) des Halteelements (4) ist in proximaler Richtung an dem Antriebsglied (2), insbesondere an dem proximalen Ende des Eingriffselements (2a), insbesondere der Aussparung (2a) des Antriebsglieds (2) abgestützt. Das proximale Ende des Antriebsglieds (2) ist in Anschlagkontakt mit dem Gehäuse (1). Der Steg (4d) des Halteelements (4) ist in der Kerbe (2e) des Antriebsglieds (2) angeordnet. Wie in der Figur 4d ersichtlich ist, wird das Blockierelement (8) von der Blockierposition in eine Freigabeposition bewegt. Der Anschlag (6e) der Aufnahmevorrichtung (6) gelangen in Anschlagkontakt mit dem Anschlag (8e) des Blockierelements (8). Durch das Einführen oder Einschrauben der Aufnahmevorrichtung (6) nimmt die Aufnahmevorrichtung (6) das Blockierelement (8) mit, wobei die Aufnahmevorrichtung (6) und das Blockierelement (8) relativ zu dem Betätigungselement (5) gedreht werden. Dabei wird die Nut-/Nockenverbindung (nicht ersichtlich) zwischen dem Blockierelement (8) und dem Gehäuse (1) gelöst. Am Ende der Einführbewegung oder der Einschraubbewegung der Aufnahmevorrichtung (6) in das Gehäuse (1) liegt der Vorsprung (5d) des Betätigungselements (5) in der axialen Flucht zu der Ausnehmung (8b) des Blockierelements (8). In der Freigabeposition des Blockierelements (8) ist das Betätigungselement (5) derart relativ zu dem Blockierelement (8) angeordnet, dass das Betätigungselement (5) entlang der Gehäuselängsachse relativ zu dem Gehäuse (1) bewegt werden kann. Die Anzeigevorrichtung (nicht ersichtlich), insbesondere eine entsprechende Ziffer, ein entsprechender Buchstabe oder ein anderes entsprechende Zeichen der Aufnahmevorrichtung (6) ist derart durch das an dem Gehäuse (1) vorgesehene Fenster (1i) ersichtlich, dass angezeigt wird, dass sich die Vorrichtung in dem entlüfteten Zustand befindet. Ferner ist der Rastarm (6d) der Aufnahmevorrichtung (6) in einer Rastöffnung (1h) des Gehäuses (1) insbesondere in einer an dem proximalen Ender der Vorrichtung angeordneten Rastöffnung (1h) des Gehäuses (1) eingerastet.

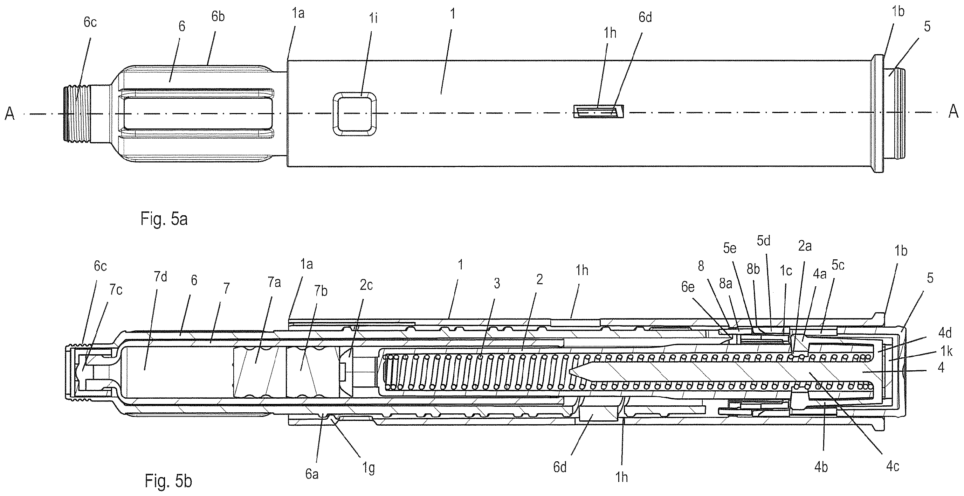

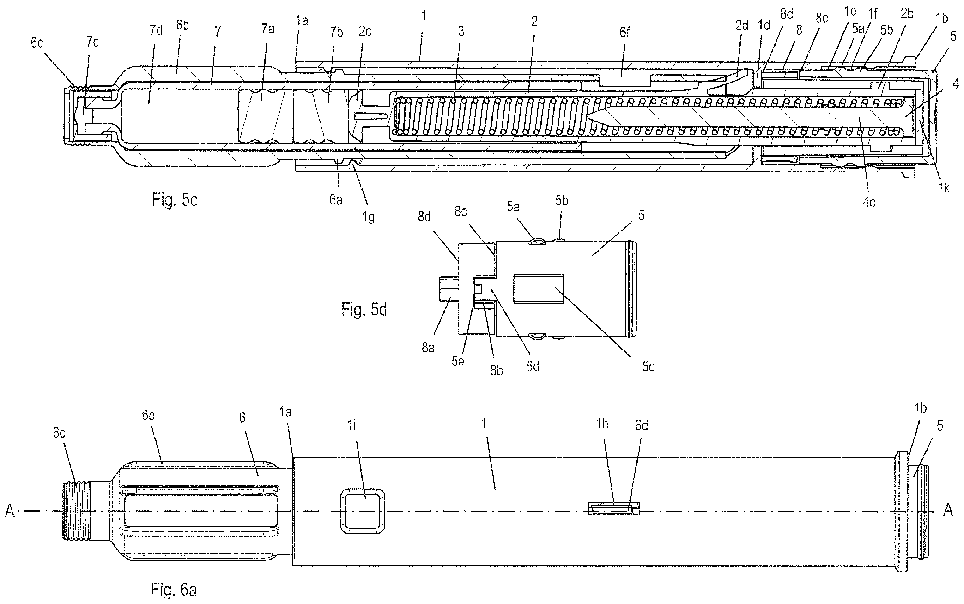

In der Figur 5b ist eine Längsschnittansicht der Vorrichtung aus der Figur 1 in einem ausgelösten Zustand dargestellt, wobei die Längsschnittansicht der in der Figur 5a eingezeichneten Schnittlinie A-A entspricht. Ferner ist in der Figur 5c eine Längsschnittansicht der Vorrichtung gemäss Figur 5b gezeigt, wobei die Längsschnittansicht gegenüber der Figur 5a um 90 Grad gedreht ist. Das Betätigungselement (5) wird relativ zu dem Gehäuse (1) in die distale Richtung gedrückt oder bewegt. Dabei gleitet der zweite Nocke (5b) des Betätigungselements (5) über die Rippe (1f) des Gehäuses (1) und bewegt sich in die distale Richtung. Wie in der Figur 5d ersichtlich ist, gelangt der Vorsprung (5d) des Betätigungselements (5) in die Ausnehmung (8b) des Blockierelements (8). Ferner gelangt die Ausnehmung (5c) des Betätigungselements (5) über das Eingriffselement (4a) des Halteelements (4), insbesondere über die Nocke (4a) des Halteelements (4). Dabei gelangt das Eingriffselement (4a) des Halteelements (4), insbesondere die Nocke (4a) des Halteelements (4) ausser Eingriff mit dem Eingriffselement (2a) des Antriebglieds (2), insbesondere der Aussparung (2a) des Antriebsglieds (2). Dabei entspannt sich der elastisch vorgespannte Arm (4b) des Halteelements (4), wobei das Eingriffselement (4a) des Halteelements (4), insbesondere die Nocke (4a) des Halteelements (4) radial nach aussen ausgelenkt wird. Dabei kann der Nocke (4a) über eine schräge Fläche, welche an dem Nocken (4a) vorgesehen ist, an einer an der Aussparung (2a) vorgesehenen schrägen Fläche entlang gleiten. Das Antriebsglied (2) ist von dem Halteelement (4) gelöst und bewegt sich aufgrund der Kraft der Feder (3) relativ zu dem Gehäuse (1) in die distale Richtung. Das Halteelement (4) gelangt aufgrund der in die proximale Richtung wirkenden Kraft der Feder (3) in proximaler Richtung in Anschlagkontakt mit dem proximalen Gehäuseanschlag (1k). Der Steg (4d) des Halteelements (4) gelangt dabei ausser Eingriff mit der Kerbe (2e) des Antriebsglieds (2).

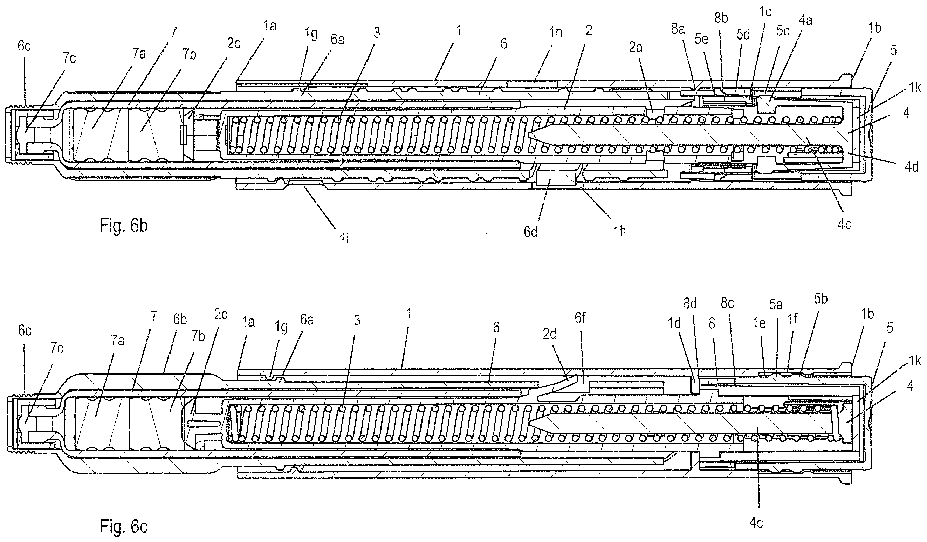

In der Figur 6b ist eine Längsschnittansicht der Vorrichtung aus der Figur 1 in einem verriegelten Zustand dargestellt, wobei die Längsschnittansicht der in der Figur 6a eingezeichneten Schnittlinie A-A entspricht. Ferner ist in der Figur 6c eine Längsschnittansicht der Vorrichtung gemäss Figur 6b gezeigt, wobei die Längsschnittansicht gegenüber der Figur 6a um 90 Grad gedreht ist. Das Antriebsglied (2) wird durch die Kraft der Feder (3) relativ zu der 2-Kammerkarpule (7) verschoben und treibt den ersten (7a) und den zweiten Stopfen (7b) innerhalb der 2-Kammerkarpule (7) an, so dass das fluide Produkt aus der ersten Kammer (7d) der 2-Kammerkarpule (7) ausgeschüttet wird. Die Feder (3) schiebt das Antriebsglied (2) so lange in der 2-Kammerkarpule (7) bis der Ausschüttungsanschlag (2b) des Antriebsglieds (2) in Anschlagkontakt mit dem Ausschüttungsanschlag (1d) des Gehäuses (1) gelangt. Der Ausschüttungsanschlag (2b) des Antriebsglieds (2) ist zwischen dem Eingriffselement (2a), insbesondere der Aussparung (2a) des Antriebselements (2) und dem proximalen Ende des Antriebglieds (2) angeordnet. Nachdem der Arm (2d) des Antriebsglieds (2) durch die Wandung des Karpulenhalters (6) weiter radial nach innen vorgespannt wird, gelangt der radial nach innen vorgespannte Arm (2d) des Antriebsglieds (2) am Ende der Ausschüttung des fluiden Produkts in die Aussparung (6f) der Aufnahmevorrichtung (6), Der Arm (2) des Antriebsglieds kann beim Einrasten, insbesondere beim Entspannen in die Aussparung (6f) der Aufnahmevorrichtung (6) ein akustisches Geräusch erzeugen, um anzuzeigen, dass die Ausschüttung beendet ist. Das Halteelement (4) ist aufgrund der in die proximale Richtung wirkenden Kraft der Feder (3) in proximaler Richtung in Anschlagkontakt mit dem proximalen Gehäuseanschlag (1k).

### Bezugszeichen:

- 1: Gehäuse
- 1a: distales Ende des Gehäuses
- 1b: proximales Ende des Gehäuses
- 1c: Vorsprung des Gehäuses
- 1d: Ausschüttungsanschlag des Gehäuses
- 1e: Längsnut des Gehäuses
- 1f: Rippe des Gehäuses
- 1g: Innengewinde des Gehäuses
- 1h: Rastöffnung
- 1h': Rastlängsnut
- 1i: Fenster
- 1j: Nocke
- 1k: proximaler Gehäuseanschlag
- 2: Antriebsglied
- 2a: Eingriffselement des Antriebsglieds, Aussparung
- 2b: Ausschüttungsanschlag des Antriebsglieds
- 2c: Haltearme
- 2d: Arm
- 2e: Kerbe
- 3: Feder
- 4: Halteelement
- 4a: Eingriffselement des Halteelements, Nocke
- 4b: Arm des Eingriffselements
- 4c: Element
- 4d: Steg
- 5: Betätigungselement
- 5a: erste Nocke des Betätigungselements
- 5b: zweite Nocke des Betätigungselements
- 5c: Ausnehmung des Betätigungselements
- 5d: Vorsprung
- 5e: Vorspungskante
- 6: Aufnahmevorrichtung
- 6a: Aussengewinde der Aufnahmevorrichtung
- 6b: Längsrippe der Aufnahmevorrichtung
- 6c: Injektionsnadeleinheitsverbindungselement
- 6d: Rastarm
- 6e: Anschlag
- 6f: Aussparung
- 7: Mehrkammerkarpule, 2-Kammerkarpule
- 7a: erste Stopfen
- 7b: zweite Stopfen
- 7c: Membran
- 7d: erste Kammer
- 7e: zweite Kammer
- 7f: Bypass
- 8: Blockierelement
- 8a: Anschlag
- 8b: Ausnehmung
- 8c: Blockierelementkante
- 8d: Blockierelementauflage

## Patentansprüche

1. Vorrichtung zur Verabreichung einer Dosis eines fluiden Produkts umfassend:
- ein Gehäuse (1) mit einem distalen (1a) und einem proximalen Ende (1b) oder Abschnitt, wobei an dem Gehäuse (1) ein Ausschüttungsanschlag (1d) einer zur verabreichenden Dosis vorgesehen ist,
- eine vorgespannte Feder (3), welche zumindest teilweise von einem Antriebsglied (2) aufgenommen ist, und das Antriebsglied (2) axial in die distale Richtung bewegbar in dem Gehäuse (1) gelagert ist, um einen in einer Karpule, insbesondere um einen in einer Mehrkammerkarpule (7) aufgenommenen Stopfen (7a, 7b) zur Verabreichung der Dosis des fluiden Produkts in die distale Richtung zu bewegen, wobei die Feder (3) an dem distalen Ende an dem Antriebsglied (2) und an dem proximalen Ende an einem Halteelement (4) abgestützt ist,
- ein Ausschüttungsanschlag (2b), welcher an dem Antriebsglied (2) vorgesehen ist, wobei der Ausschüttungsanschlag (2b) des Antriebsglieds (2) in Anschlagkontakt mit dem Ausschüttungsanschlag (1d) des Gehäuses (1) zur Verabreichung der Dosis des fluiden Produkts gelangbar ist, und der Ausschüttungsanschlag (2b) des Antriebsglieds (2) axial in die proximale Richtung versetzt zu dem Eingriffselement (2a) des Antriebsglieds (2) an dem Antriebsglied (2) angeordnet ist, **dadurch gekennzeichnet, dass**
- das Halteelement (4) zum Halten des mit einer Kraft der Feder (3) beaufschlagten Antriebsglieds (2) vorgesehen ist, wobei in einem Ausgangszustand der Vorrichtung das Antriebsglied (2) durch einen Eingriff zwischen einem an dem Halteelement (4) vorgesehenen Eingriffselement (4a) und einem an dem Antriebsglied (2) vorgesehenen Eingriffselement (2a) in einer proximalen Position haltbar ist, wobei in einem ausgelösten Zustand der Vorrichtung das Eingriffselement (4a) des Halteelements (4) ausser Eingriff mit dem Eingriffselement (2a) des Antriebsglieds (2) ist, wobei
- zumindest im Ausgangszustand der Vorrichtung das proximale Ende des Antriebsglieds in Anschlagkontakt mit dem Gehäuse ist und an dem Gehäuse ein Vorsprung vorgesehen ist, um eine relative Bewegung des Halteelements zu dem Gehäuse in distaler Richtung zu begrenzen,
- wobei in dem Ausgangszustand der Vorrichtung das Halteelement (4) in proximaler Richtung von dem proximalen Ende oder Abschnitt des Gehäuses (1b, 1k) beabstandet ist

2. Vorrichtung nach Anspruch 1,
**dadurch gekennzeichnet, dass** der Ausschüttungsanschlag (2b) des Antriebsglieds (2) in Umfangsrichtung um 90 Grad versetzt zu dem Eingriffselement (2a) des Antriebsglieds (2) an dem Antriebsglied (2) angeordnet ist.

3. Vorrichtung nach Anspruch 1 oder 2,
**dadurch gekennzeichnet, dass** das Antriebsglied (2) hülsenförmig ausgebildet ist.

4. Vorrichtung nach einem der vorhergehenden Ansprüche,
**dadurch gekennzeichnet, dass** in dem Ausgangszustand der Vorrichtung das proximale Ende des Antriebsglieds (2) in Anschlagkontakt mit dem Gehäuse (1) ist.

5. Vorrichtung nach einem der vorhergehenden Ansprüche,
**dadurch gekennzeichnet, dass** das Halteelement (4) einen Steg (4d) aufweist, welcher zumindest in dem Ausgangszustand der Vorrichtung in einer an dem proximalen Ende des Antriebsglieds (2) vorgesehenen Kerbe (2e) angeordnet ist.

6. Vorrichtung nach einem der vorhergehenden Ansprüche,
**dadurch gekennzeichnet, dass** der Aussendurchmesser des Antriebsglieds (2) am distalen Ende des Antriebsglieds (2) und am proximalen Endes des Antriebglieds (2) unterschiedliche ist, wobei der Innendurchmesser des Antriebsglieds (2) am distalen und am proximalen Ende des Antriebsglieds (2) gleich ist.

7. Vorrichtung nach Anspruch 6,
**dadurch gekennzeichnet, dass** der Aussendurchmesser des Antriebglieds (2) grösser am proximalen Ende als am distalen Ende des Antriebsglieds (2) ist, wobei der Aussendurchmesser des distalen Ende des Antriebsglieds (2) derart ausgebildet ist, dass das distale Ende des Antriebsglieds (2) in die Karpule, insbesondere in die Mehrkammerkarpule (7) ragt, wenn der Ausschüttungsanschlag (2b) des Antriebsglieds (2) in Anschlagkontakt mit dem Ausschüttungsanschlag (1d) des Gehäuses (1) ist.

8. Vorrichtung nach einem der vorhergehenden Ansprüche,
**dadurch gekennzeichnet, dass** das Eingriffselement (4a) des Halteelements (4) als Nocke (4a) und das Eingriffselement (2a) des Antriebglieds (2) als Aussparung (2a) ausgebildet ist.

9. Vorrichtung nach einem der vorhergehenden Ansprüche,
**dadurch gekennzeichnet, dass** ferner ein Betätigungselement (5) zum Auslösen der Vorrichtung vorgesehen ist, welches relativ zu dem Gehäuse (1) bewegbar ist.

10. Vorrichtung nach Anspruch 9,
**dadurch gekennzeichnet, dass** das Betätigungselement (5) eine Ausnehmung (5c) aufweist, in welche das Eingriffselement (4a) des Halteelements (4) in dem ausgelösten Zustand der Vorrichtung ragbar ist.

11. Vorrichtung nach einem der vorhergehenden Ansprüche,
**dadurch gekennzeichnet, dass** ferner eine Aufnahmevorrichtung (6) zur Aufnahme einer Mehrkammerkarpule (7) vorgesehen ist, wobei die Aufnahmevorrichtung (6) relativ zum Gehäuse (1) drehbar gelagert ist, wobei durch Drehung der Aufnahmevorrichtung (6) die Mehrkammerkarpule (7) abmischbar ist.

## Claims

1. Apparatus for administering a dose of a fluid product including:
- a housing (1) having a distal end (1a) and a proximal end (1b) or portion, wherein provided on the housing (1) is a dispensing abutment (1d) of a dose to be administered,
- a prestressed spring (3) which is at least partially accommodated by a drive member (2) and the drive member (2) is mounted in the housing (1) moveably axially in the distal direction in order to move a plug (7a, 7b) accommodated in a carpule, in particular in a multi-chamber carpule (7), for administering the dose of the fluid product, in the distal direction, wherein the spring (3) is supported at the distal end at a drive member (2) and at the proximal end at a holding element (4),
- a dispensing abutment (2b) provided on the drive member (2), wherein the dispensing abutment (2b) of the drive member (2) can be brought into abutting contact with the dispensing abutment (1d) of the housing (1) for administering the dose of the fluid product, and the dispensing abutment (2b) of the drive member (2) is arranged displaced axially in the proximal direction relative to the engagement element (2a) of the drive member (2) at the drive member (2), **characterised in that**
- the holding element (4) is provided for holding the drive member (2) acted upon with a force of the spring (3), wherein in a starting condition of the apparatus the drive member (2) can be held in a proximal position by an engagement between an engagement element (4a) provided on the holding element (4) and an engagement element (2a) provided on the drive member (2), wherein in the triggered condition of the apparatus the engagement element (4a) of the holding element (4) is out of engagement with the engagement element (2a) of the drive member (2), wherein
- at least in the starting condition of the apparatus the proximal end of the drive member is in abutting contact with the housing and provided on the housing is a projection to limit a relative movement of the holding element with respect to the housing in the distal direction,
- wherein in the starting condition of the apparatus the holding element (4) is spaced in the proximal direction from the proximal end or portion of the housing (1b, 1k).

2. Apparatus according to claim 1
**characterised in that** the dispensing abutment (2b) of the drive member (2) is arranged on the drive member (2) displaced in the peripheral direction through 90 degrees relative to the engagement element (2a) of the drive member (2).

3. Apparatus according to claim 1 or claim 2
**characterised in that** the drive member (2) is of a sleeve-shaped configuration.

4. Apparatus according to one of the preceding claims
**characterised in that** in the starting condition of the apparatus the proximal end of the drive member (2) is in abutting contact with the housing (1).

5. Apparatus according to one of the preceding claims
**characterised in that** the holding element (4) has a leg (4d) which at least in the starting condition of the apparatus is arranged in a notch (2e) provided at the proximal end of the drive member (2).

6. Apparatus according to one of the preceding claims
**characterised in that** the outside diameter of the drive member (2) is different at the distal end of the drive member (2) and at the proximal end of the drive member (2), wherein the inside diameter of the drive member (2) is the same at the distal and proximal ends of the drive member (2).

7. Apparatus according to claim 6
**characterised in that** the outside diameter of the drive member (2) is larger at the proximal end than at the distal end of the drive member (2), wherein the outside diameter of the distal end of the drive member (2) is such that the distal end of the drive member (2) projects into the carpule, in particular into the multi-chamber carpule (7), when the dispensing abutment (2b) of the drive member (2) is in abutting contact with the dispensing abutment (1d) of the housing (1).

8. Apparatus according to one of the preceding claims
**characterised in that** the engagement element (4a) of the holding element (4) is in the form of a cam (4a) and the engagement element (2a) of the drive member (2) is in the form of a recess (2a).

9. Apparatus according to one of the preceding claims
**characterised in that** there is further provided an actuating element (5) for triggering the apparatus, the actuating element being moveable relative to the housing (1).

10. Apparatus according to claim 9
**characterised in that** the actuating element (5) has a recess (5c) into which the engagement element (4a) of the holding element (4) can project in the triggered condition of the apparatus.

11. Apparatus according to one of the preceding claims
**characterised in that** there is further provided a receiving device (6) for receiving a multi-chamber carpule (7), wherein the receiving device (6) is mounted rotatably relative to the housing (1), wherein the multi-chamber carpule (7) can be mixed by rotation of the receiving device (6).

## Revendications

1. Dispositif pour l'administration d'une dose d'un produit fluide, comprenant :
- un boîtier (1) avec une extrémité ou portion distale (la) et une extrémité ou portion proximale (1b), dans lequel, sur le boîtier (1), est prévue une butée de distribution (1d) d'une dose à administrer,
- un ressort précontraint (3), qui est logé au moins partiellement par un organe d'entraînement (2) et l'organe d'entraînement (2) est logé axialement de manière mobile dans la direction distale dans le boîtier (1), afin de déplacer un obturateur (7a, 7b) logé dans une cartouche, plus particulièrement dans une cartouche multi-chambres (7), pour l'administration de la dose de produit fluide dans la direction distale, dans lequel le ressort (3) s'appuie, au niveau de l'extrémité distale, contre l'organe d'entraînement (2) et, au niveau de l'extrémité proximale, contre un élément de maintien (4),
- une butée de distribution (2b), qui est prévue sur l'organe d'entraînement (2), dans lequel la butée de distribution (2b) de l'organe d'entraînement (2) peut être mise en contact de butée avec la butée de distribution (1d) du boîtier (1) pour l'administration de la dose de produit fluide et la butée de distribution (2b) de l'organe d'entraînement (2) est disposée sur l'organe d'entraînement (2) de manière décalée axialement dans la direction proximale par rapport à l'élément d'emboîtement (2a) de l'organe d'entraînement (2), **caractérisé en ce que**
- l'élément de maintien (4) est conçu pour le maintien de l'organe d'entraînement (2) sollicité par une force du ressort (3), dans lequel, dans un état initial du dispositif, l'organe d'entraînement (2) peut être maintenu dans une position proximale par un emboîtement entre un élément d'emboîtement (4a) prévu sur l'élément de maintien (4) et un élément d'emboîtement (2a) prévu sur l'organe d'entraînement (2), dans lequel, dans un état déclenché du dispositif, l'élément d'emboîtement (4a) de l'élément de maintien (4) est déboîté de l'élément d'emboîtement (2a) de l'organe d'entraînement (2), dans lequel
- au moins dans l'état initial du dispositif, l'extrémité proximale de l'organe d'entraînement est en contact de butée avec le boîtier et, sur le boîtier, est prévue une saillie permettant de limiter un mouvement relatif de l'élément de maintien par rapport au boîtier dans la direction distale,
- dans lequel, dans l'état initial du dispositif, l'élément de maintien (4) est distant, dans la direction proximale, de l'extrémité ou de la portion proximale du boîtier (1b, 1k)

2. Dispositif selon la revendication 1,
**caractérisé en ce que** la butée de distribution (2b) de l'organe d'entraînement (2) est disposée, sur l'organe d'entraînement (2), de manière décalée dans la direction circonférentielle de 90 degrés par rapport à l'élément d'emboîtement (2a) de l'organe d'entraînement (2).

3. Dispositif selon la revendication 1 ou 2,
**caractérisé en ce que** l'organe d'entraînement (2) présente une forme de manchon.

4. Dispositif selon l'une des revendications précédentes,
**caractérisé en ce que**, dans l'état initial du dispositif, l'extrémité proximale de l'organe d'entraînement (2) est en contact de butée avec le boîtier (1).

5. Dispositif selon l'une des revendications précédentes,
**caractérisé en ce que** l'élément de maintien (4) comprend une nervure (4d) qui est disposée, au moins dans l'état initiale du dispositif, dans une encoche (2e) prévue au niveau de l'extrémité proximale de l'organe d'entraînement (2).

6. Dispositif selon l'une des revendications précédentes,
**caractérisé en ce que** le diamètre extérieur de l'organe d'entraînement (2) est différent à l'extrémité distale de l'organe d'entraînement (2) et à l'extrémité proximale de l'organe d'entraînement (2), dans lequel le diamètre intérieur de l'organe d'entraînement (2) est le même à l'extrémité distale et à l'extrémité proximale de l'organe d'entraînement (2).

7. Dispositif selon la revendication 6,
**caractérisé en ce que** le diamètre extérieur de l'organe d'entraînement (2) est plus grand à l'extrémité proximale qu'à l'extrémité distale de l'organe d'entraînement, dans lequel le diamètre extérieur de l'extrémité distale de l'organe d'entraînement (2) est conçu de façon à ce que l'extrémité distale de l'organe d'entraînement (2) dépasse dans la cartouche, plus particulièrement dans la cartouche multi-chambres (7), lorsque la butée de distribution (2b) de l'organe d'entraînement (2) est en contact de butée avec la butée de distribution (1d) du boîtier (1).

8. Dispositif selon l'une des revendications précédentes,
**caractérisé en ce que** l'élément d'emboîtement (4a) de l'élément de maintien (4) est conçu comme une came (4a) et l'élément d'emboîtement (2a) de l'organe d'entraînement (2) est conçu comme un évidement (2a).

9. Dispositif selon l'une des revendications précédentes,
**caractérisé en ce que**, en outre, un élément d'actionnement (5) est prévu pour le déclenchement du dispositif, qui est mobile par rapport au boîtier (1).

10. Dispositif selon la revendication 9,
**caractérisé en ce que** l'élément d'actionnement (5) comprend un évidement (5c) dans lequel l'élément d'emboîtement (4a) de l'élément de maintien (4) peut dépasser du dispositif dans l'état déclenché.

11. Dispositif selon l'une des revendications précédentes,
**caractérisé en ce que**, en outre, un dispositif de logement (6) est prévu pour le logement d'une cartouche multi-chambres (7), dans lequel le dispositif de logement (6) est logé de manière mobile par rapport au boîtier (1), dans lequel la rotation du dispositif de logement (6) permet de mélanger la cartouche multi-chambres (7).
